# EUROPEAN PATENT SPECIFICATION

(11) **EP 3 808 734 B1**
(45) Date of publication and mention of the grant of the patent: **30.03.2022**
(21) Application number: 20153990.5
(22) Date of filing: 28.01.2020
(51) Int. Cl.: C07C 303/22, C07C 303/44, C07C 309/14

(54) **THE METHOD AND PRODUCTION SYSTEM FOR FULLY RECOVERING AND TREATING TAURINE MOTHER LIQUOR**
VERFAHREN UND PRODUKTIONSSYSTEM ZUR VOLLSTÄNDIGEN RÜCKGEWINNUNG UND BEHANDLUNG VON TAURIN-MUTTERLAUGE
PROCÉDÉ ET SYSTÈME DE PRODUCTION POUR RÉCUPÉRER ET TRAITER ENTIÈREMENT UNE LIQUEUR MÈRE TAURINE

(30) Priority: 16.10.2019 CN 201910986942
(43) Date of publication of application: 21.04.2021
(73) Proprietor: Qiangjiang Yongan Pharmaceutical Co., Ltd., Qianjiang, Hubei 433100 (CN)
(72) Inventor: Chen, Yong, Qianjiang, Hubei 433100 (CN); Fang, Xiquan, Qianjiang, Hubei 433100 (CN); Li, Shaobo, Qianjiang, Hubei 433100 (CN); Liu, Feng, Qianjiang, Hubei 433100 (CN); Jiang, Xiaojun, Qianjiang, Hubei 433100 (CN); Zhou, Wei, Qianjiang, Hubei 433100 (CN)
(74) Representative: Gulde & Partner

(56) References cited:
- US-A1- 2015 299 114
- US-A1- 2015 299 144

## Description

### Field of Invention

The invention relates to a production method in a chemical synthesis taurine process, in particular to a method for removing impurities and recycling the mother liquor produced by the production of taurine by an ethylene oxide method.

### Background of the Invention

The chemical name of taurine which is 2-aminoethanesulfonic acid is the most abundant sulfur-containing free amino acid in body cell. The chemical synthesis route of taurine mainly includes ethylene oxide method and ethanolamine method. Among them, the preparation of the ethylene oxide process comprises three steps:
(1) Utilizing ethylene oxide as a starting material, an addition reaction of ethylene oxide and sodium hydrogen sulfite generates sodium isethionate;
   Main Reaction:

      *CH₂CH₂O*+ *NaHSO₃→ HOCH₂CH₂SO₃Na*

      *HOCH₂CH₂SO₃Na* + *NH₃ → H₂NCH₂CH₂SO₃Na* + *H₂O*

      *2H₂NCH₂CH₂SO₃Na* + *H₂SO₄ → 2H₂NCH₂CH₂SO₃H* + *Na₂SO₄*
   Addition side reaction:

      *CH₂CH₂O* + *H₂O → HOCH₂CH₂OH*
(2) Aminolysis of sodium isethionate to generate sodium taurate;
   Ammonialysis side reaction:

   *2HOCH₂CH₂SO₃Na* + *NH₃ → HN(CH₂CH₂SO₃Na)₂* + *2H₂O*

   *3HOCH₂CH₂SO₃Na* + *NH₃ → N (CH₂CH₂SO₃Na)₃* + *3H₂O*
(3) Taurine is generated through acidification, for example hydrochloric acid, preferably sulfuric acid, is neutralized to generate taurine and the inorganic salt.

By-products are inevitably produced in the said addition and synthesis reactions, including ethylene glycol, ethylene glycol polymers and so on. The aminolysis reaction is a reversible reaction. About more than 20% of sodium isethionate will enter the next process with the production system. After the aminolysis reaction the ammonia solution is neutralized by sulfuric acid, the mother liquor is separated and concentrated for 1-3 times, and then the last mother liquor is generated. The impurities in the last mother liquor mainly include taurine, sodium isethionate, sodium sulfate, sodium iminodisulfonate, ethylene glycol and polyethylene glycol, and trace metal ions, which are highly polluting emissions. When the existing production method adopts the mother liquor circulation, the cumulative increase of by-products will occur. When the by-product reaches a threshold, it can only be solved by discharging part of the mother liquor, but resulting in waste and pollution.

Chinese patents CN101508657, CN10158658, CN10158659 and CN101486669 describe a process of generating taurine and sodium sulfate by neutralizing sodium taurate by sulfuric acid. The crude taurine can be easily generated by filtering the crystal suspension liquor after cooling. However, the discarded mother liquor still contains taurine, sulfate and other organic impurities.

On the use of mother liquor, Research on Taurine Ammonolysis Process, published in Issue 5, Volume 44 of Shandong Chemical Industry, 2015, (Author: Liu Fuming, Xie Limin), specifying in detail the process of taurine reaction, and the other organic impurities in the reaction, such as ethylene glycol and polyethylene glycol, analyzes the effect of mother liquor application on yield. The higher the content of mother liquor in the reaction system, the higher the product yields. In the actual production process, the amount of mother liquor cannot be infinitely increased. As the content of mother liquor increases, by-products in the reaction system increase greatly, and the output of the last mother liquor in the production process can only meet the maximum 9.0% (v/v) set of dosage. Considering comprehensive production costs and yield quality, it is most appropriate to choose the mother liquor content of 6.3% - 8.3% (v/v). Therefore, the removal of impurities in the mother liquor is a prerequisite for increasing the application of the mother liquor, otherwise the increase in the amount of application will cause more by-products in production and the production will be more unstable.

Chinese patent CN107056659A describes a method for neutralizing sodium taurate by ion exchange to generate taurine, and then recycling the mother liquor to further increase the yield. The process route mainly avoids the generation of sulfate, recycles sodium atoms therein, and greatly saves raw materials such as sulfuric acid and sodium hydroxide, wherein the extracted mother liquor is returned to the aminolysis reaction as a raw material. However, the process does not avoid the occurrence of side reactions such as addition and synthesis, and the mother liquor still needs to be subjected to removing impurity treatment.

With regard to the removing impurity treatment of the mother liquor, Chinese Patent CN105732440 discloses a method for producing taurine by fully recovering the mother liquor, which mainly removes impurities by neutralization in the second stage to obtain crude taurine, and the mother liquor is subjected to pressure filtration and catalysis to further remove sulfuric acid. After that, the sodium is reused in the synthesis section. Among them, the removal effect of ethylene glycol and other organic polymers therein by the second-stage neutralization and removing impurity is limited.

In summary, although the current taurine preparation process is relatively mature, there are still many deficiencies in the separation and purification of taurine and the recycling of the mother liquor. In particular, the viscosity of organic impurities in the final liquid mother liquor produced by the system for producing taurine is high, and it is a difficult problem to separate them in large-scale production. It is urgent to seek an effective solution.

US 2015/0299114 A1 discloses a cyclic process for the production of taurine from alkali isethionate and alkali vinyl sulfonate in a high overall yield of greater than 95% by continuously converting the byproducts of the ammonolysis reaction, sodium ditaurinate and sodium tritaurinate, to sodium taurinate. Sodium sulfate and residual taurine in the crystallization mother liquor are separated by converting taurine into a highly soluble form of sodium taurinate or ammonium taurinate while selectively crystallizing sodium sulfate.

### Summary of Invention

The purpose of the invention is to provide a pretreatment process for the last mother liquor of taurine, which can effectively reduce the viscosity of the last mother liquor, remove some impurities at the same time, and improve greatly the recovery treatment efficiency.

Further, this invention provides an impurity-removal and mother liquor recovery method using the said pretreatment method to achieve full recovery of the mother liquor.

The invention is defined by the appended claims. The description that follows is subjected to this limitation. Any disclosure lying outside the scope of said claims is only intended for illustrative as well as comparative purposes.

After extensive research and experimentation, the inventor unexpectedly discovered a method for removing and recovering taurine mother liquor, adding a pretreatment process to the mother liquor in the existing ethylene oxide process for preparing taurine. The viscosity of the last mother liquor is lowered, and then the subsequent decolorization and removing impurity treatment is conducted, the liquor can be efficiently recycled finally.

A method for fully recovering and treating taurine mother liquor applied in ethylene oxide production process of taurine, comprises the following steps:
(a) adding alkali firstly and then acid to a last mother liquor of taurine to form salt in the solution, or directly adding salt formed by the reaction of alkali with acid to the last mother liquor of taurine, wherein the alkali is any one or the mixture of sodium hydroxide, potassium hydroxide, lithium hydroxide, sodium carbonate, potassium carbonate and lithium carbonate, and the acid is sulfuric acid, hydrochloric acid, or an organic acid;
(b) concentrating and crystallizing the material collected in step (a); and
(c) obtaining a clear last mother liquor of taurine through filtration.

Preferably, the reaction temperature of the said step (a) is 50°C - 95°C, the pH value after acid addition is 7.0-10.5, the reaction temperature is preferably 50°C - 75°C, and the pH value is 8.5-10.0.

Preferably, the amount of the base added in the said step (a) is from 5% to 50%, preferably from 15% to 35%, of the volume of the said last mother liquor of the taurine.

Further, the said alkali in step (a) is any one of sodium hydroxide, potassium hydroxide, lithium hydroxide, sodium carbonate, potassium carbonate, lithium carbonate and so on, or the mixture of any two or more thereof; the said acid may be sulfuric acid, hydrochloric acid, organic acid, etc., or the salt formed by the reaction of the said alkali with the said added acid. For example, the said sodium hydroxide reacts with sulfuric acid to form sodium sulfate, and potassium hydroxide reacts with sulfuric acid to form potassium sulfate, etc., wherein the amount of the salt-added substance for 1 m³ of the last mother liquor is 530mol-5300mol: it is preferred to add liquid alkali and sulfuric acid successively, according to the principle of chemical acid-alkali reaction, the sodium is formed into sodium taurate, and then sodium sulfate is formed, so that the advantage of the selection is that it will not increase the production system. New impurities are more conducive to recycling applications.

Preferably, in step (b), water is condensed and evaporated by 30-60%, preferably 35%-45%, and the concentration times are about 1.4-2.5, preferably 1.5-1.8 times, to obtain the crystallization solution.

Preferably, in step (b), the mother liquor is concentrated, cooled and crystallized.

Subsequently, the mother liquor after the said pretreatment is further subjected to decolorization and removing impurities treatment by the activated carbon, and the last mother liquor of the taurine generated in step (c) is decolorized by adding activated carbon under cooling condition, and the pH value is adjusted to 9.0-10.5 by adding alkali, and then filtered.

Preferably, the said temperature-lowering condition means that the temperature of the production system is lower than the temperature of the previous process, and the system processing temperature is controlled among 15-25°C, more preferably 18-22°C;

Preferably, the activated carbon decolorization and the alkali-filtered outlet liquid are subjected to ammonia-mixing treatment, and the solid liquid separation is performed to generate the mother liquid after the impurity removal, and all the mother liquid is returned to the aminolysis step.

After the activated carbon is decolorized, when ammonia-mixing treatment is required, add liquid ammonia or ammonia to the outlet liquid, and the mass to volume ratio of ammonia is 15g/100 ml (15%) or more, preferably 17-19g/ 100 ml (17-19%) to remove impurities such as salt.

After the said treatment, the by-products in the reaction process can be removed, and because the viscosity of the by-products is high, separation is difficult, which is unfavorable for industrial production. The method combines the natures of solubility of taurine, sulfate, impurities and the like at different temperatures, concentrations and pH conditions, controlling the temperature, concentration and pH value of the reaction in each step, so that the impurities can be precipitated, and the active ingredients are not precipitated, thus creating conditions for the separation of impurities and active ingredients; and then utilizing the reaction, the solution reacts with the liquid alkali to make the substance in the solution exist as a sodium salt, and then neutralizes it by using sulfuric acid, which will form sodium sulfate, and then crystallize it by concentration, therefore sodium sulfate and impurities are precipitated, and the generated sodium sulfate acts indirectly as filter aid. The function achieves the purpose of rapid separation and facilitates the embodiment of industrialization.

The impurity-removal and mother liquid recovery in ethylene oxide process taurine production process comprises the following steps:
S1: ethylene oxide reacts with sodium hydrogen sulfite solution to generate sodium isethionate;
S2: sodium isethionate generated by S1, the mother liquor after treatment, ammonia water is mixed to generate the reaction liquid, and then ammonia is absorbed to a certain degree of concentration, and an aminolysis reaction is carried out under the action of catalyst, and the sodium taurate solution is generated by evaporation and concentration;
S3: the sodium taurate solution generated in S2 is prepared at a certain degree of concentration, and the taurine solution can be generated by using acidic cation exchange resin, and then concentrate and crystallize it to generate the crude taurine and mother liquor; or add sulfuric acid to neutralize to pH 7.0-8.5, the taurine crystallization solution is obtained, and the crude taurine and mother liquor are generated after cooling and crystallizing;
S5. add a certain amount of alkali to the last mother liquor of taurine collected in S4, and stir it evenly, preferably liquid alkali. A certain amount of acid shall be added further thereto to achieve the pH value of 7.0-10.5, and the reaction temperature is controlled from 50°C to 75°C. Specifically, the acid is preferably sulfuric acid, and the pH value is adjusted to 8.5-10, preferably 9.0-9.5.
S6: transfer the material collected by S5 to a concentrator system for concentration, and the water is concentrated and evaporated by 30-60%, preferably 35%-45%, and the concentration times are about 1.4-2.5, preferably 1.5-1.8, to generate the crystallization solution. Preferably, the water is evaporated by 40%-42%, and the concentration ratio is preferably from 1.65 to 1.72 times.
S7: transfer the material collected by S6 to cooling crystallization tank, and cool it down to 65-99°C, preferably 72-78°C, and then filter into a plate and frame (the other types of filtration devices are also permitted) to generate the transparent solution.
S8: the solution collected by S7 is cooled to 15-25°C, preferably 18-22°C, and then a certain amount of activated carbon is added, and the liquid alkali is added to achieve the pH value of 9.0-10.5, preferably the temperature is lowered to the pH value of 9.5-10 and then passed through the plate and frame, micropore filter or other types of filtration devices to separate the mother liquor.
S9: the mass ratio of the mother liquor collected by S8 to liquid ammonia or ammonia to ammonia content under cooling conditions is greater than 15g/100ml (15%), preferably 17-19g/100ml (17-19%) ), a large amount of salt and other impurities will be precipitated, and then filter it with a vane filter or a sealed plate to generate the clear mother liquid, and the generated mother liquid can be returned to the step of S2, and the aminolysis step is used.

It should be noted that, the sodium isethionate generated by S1 can be concentrated and crystallized and dried to generate the corresponding solid, or the mixed liquid generated by the reaction can be directly mixed with the ammonia water of S2 without treatment after the direct reaction is completed. The ammonia concentration in the reaction solution after ammonia absorption is 20-28wt% (weight percent).

Specifically, the concentration of the sodium hydrogen sulfite solution in S1 is 9-36wt%, and the ratio of the amount of the substance of sodium hydrogen sulfite to ethylene oxide is 1:0.95-1.

Specifically, the catalyst in S2 is any one of sodium hydroxide, potassium hydroxide, lithium hydroxide, sodium carbonate, potassium carbonate, lithium carbonate, or the mixture of any two or more, and the temperature of the aminolysis reaction is 150-290°C, pressure is 10-25MPa. Preferably, the alkali added to S5 is the same as the catalyst in S2, avoiding new impurities being generated.

Specifically, when the sodium taurate is treated with the acidic cation exchange resin column in S3, the concentration of the sodium taurate solution is 15%-35%, preferably 18%-20%. When sodium taurate is treated with sulfuric acid in S3, the concentration of sodium taurate solution is 25%-40%, preferably 32%-38%.

The crude taurine in S3 needs to be dissolved in water, activated carbon, etc., then decolorized and filtered, cooled and crystallized, and dried after centrifugation to generate the finished taurine. The fine mother liquor after centrifugation can be reused in the preparation of sodium taurate solution or taurine decolorization ingredients.

Specifically, the mother liquor in S4 is concentrated and crystallized several times, and can be concentrated once or twice, respectively, to generate crude products of two or three times, and the generated mother liquor of the taurine can be the second mother liquor or the third mother liquors. When S3 is treated with an acidic cation exchange resin column for the treatment of sodium taurate, it is preferred that the last mother liquor of taurine is the second mother liquor; in S3, when sulfuric acid is used to treat sodium taurate, the preferred last mother liquor is the third mother liquors.

A production system for fully recovering and treating the taurine mother liquor, which reduces the filtration resistance of the last mother liquor by a pretreatment device comprising a reaction device and/or a concentration device connected in series, cooling and crystallization device and the first filtration device, can be used for applying the afore-mentioned method.

Preferably, the said discharge port of the pretreatment device is connected to an activated carbon decolorization and removing impurity device, and the said activated carbon decolorization and removing impurity device comprises a decolorization tank and the second filter device, and the decolorization tank is equipped with a feed port for adding activated carbon and alkali. There is also a cooling mechanism for reducing the temperature inside the tank.

Preferably, the said activated carbon decolorization and removing impurity device comprises a decolorization tank, an inlet frame filter pump, a plate and frame filter, a transfer tank, a precision filtration pump, a precision filter and a receiving storage tank which are sequentially connected.

Preferably, the said discharge port of the activated carbon decolorization and removing impurity device is connected to an ammonia-mixing desalination device, and the ammonia-mixing device includes an ammonia-mixing reaction tank and a closed filtration device provided with a circulation passage.

Preferably, the said ammonia-mixing reaction tank is equipped with an ammonia inlet, a feed port and a discharge port, and the said terminal discharge port of the ammonia-mixing reactor is connected to a feed port of the said sealed filter device by a pump, and the pump is equipped with a rotary discharge valve for discharging the filtered clear material.

Preferably, the said first filtering device is a plate and frame filter.

Preferably, the reaction device and the temperature-lowering crystal slurry device of the said pretreatment device are respectively equipped with a temperature lowering mechanism for lowering the temperature inside the tank.

Preferably, the said cooling mechanism is an externally disposed water circulation condensation layer, and the condensation layer is provided with a cooling water inlet valve and a cooling water outlet valve.

This invention and the current technology have the following advantages and beneficial effects:
1. According to the method used in the this invention, the salt is added to the last mother liquor of taurine, or the last mother liquor of taurine is treated with alkali and acid to realize the phenomenon that impurities are precipitated by at different temperatures, concentrations and pH, and the active ingredients are not precipitated. And then the added or generated salt forms a bridge with the precipitated impurities, reduces the filtration resistance, and also reduces the viscosity of the mother liquor, solving the problem of separating impurities, greatly shortening the time of impurity removal, and thus removing effectively more impurities in the mother liquor, and further removing the salt by ammonia-mixing treatment to obtain the pure taurine mother liquor, thereby realizing recovery of the mother liquor to improve product yield.
2. The activated carbon decolorization and removing impurity device used in this invention is completed under the condition of cooling, and the activated carbon adsorbs a certain amount of impurities such as ethylene glycol, metal ions and a small amount of organic substances, so that the impurities in the taurine mother liquor are removed more thoroughly.
3. The impurity recovery production system of this invention adopts an optimized design and utilizes an effective removing impurity device to remove all kinds of impurities contained in the last mother liquor. It is efficient, complete, simple in operation and low in operation cost.

### Brief Description of the Drawings

In order to illustrate more clearly the technological solution in the embodiment, the attached figures which are needed for the description of the embodiments will be introduced one by one. Outstandingly, the following attached figures in descriptions are the embodiments of this invention. As for the common technicians in this technological field, they can obtain the other attached figures according to these figures without offering the creative works.
Figure 1: The process flow chart of the last mother liquor pretreatment of the embodiment of this invention;
Figure 2: The process flow chart for producing taurine by the ethylene oxide method of the application embodiment of this invention; wherein, for the neutralization process of the taurine resin, the decolorization and removing impurities with the activated carbon and the ammonia-mixing treatment are performed after the pretreatment;
Figure 3: The process flow chart for producing taurine by the ethylene oxide method of the application embodiment of the present invention; wherein, for the taurine sulfuric acid neutralization process, the decolorization and removing impurities with the activated carbon and the ammonia-mixing treatment are performed after the pretreatment;
Figure 4: Schematic diagram of a mother liquor pretreatment apparatus;
Figure 5: Schematic diagram of the activated carbon decolorization and removing impurity device;
Figure 6: Schematic diagram of the ammonia-mixing and desalination device.

### Detailed Description

The details of this invention are further described below with reference to the attached drawings and specific embodiments. The embodiment raised is only for explaining this invention, but not limiting the scope of this invention.

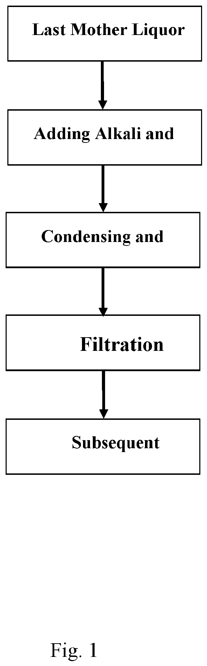

As shown in Figure 1, this invention provides a method for removing the impurities and recovering the taurine mother liquor and adding the pretreatment process to the last mother liquor treatment in the existing ethylene oxide process for preparing the taurine process. Namely, the last mother liquor of taurine is added with alkali and acid successively, and then it is concentrated, crystallized and filtered. The viscosity of the last mother liquor is reduced firstly to generate the transparent last mother liquor of taurine, and then conduct the subsequent decolorization and impurity removal treatment, so the last mother liquor could be recycled and utilized efficiently.

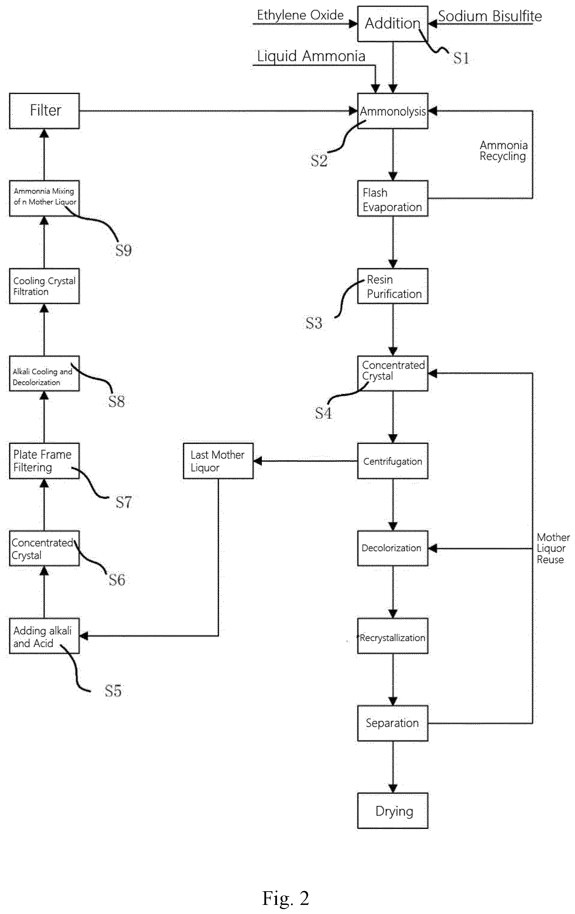

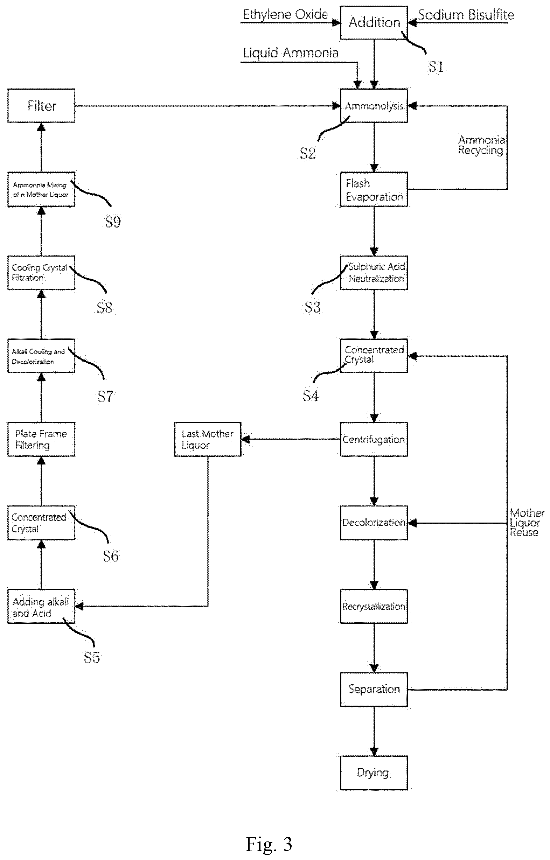

As shown in Figures 2 and 3, the said pretreatment process is applied to the process of preparing taurine by an ethylene oxide process.

S1: ethylene oxide reacts with sodium hydrogen sulfite solution to generate sodium isethionate; in this step, impurities such as ethylene glycol and polyethylene glycol are generated.

S2: sodium isethionate generated by S1, mother liquor after impurity removal treatment, and ammonia water are mixed to generate the reaction solution, and then ammonia is absorbed to a certain concentration, and the aminolysis reaction is carried out under the action of catalyst. After the reaction is completed, flash evaporation is performed to make excessive ammonia be discharged out of the reaction liquid, and then the discharged ammonia is recycled and utilized as the raw material for ammonolysis reaction. Then, it is concentrated by evaporation to generate the sodium taurate solution; and the ethylene glycol by-product in the sodium isethionate solution produced in the step S1 is converted into organic impurities such as polyether alcohols. Since the aminolysis reaction is a reversible reaction, according to the chemical equilibrium theory, the presence of sodium ditaurate or sodium tritaurate can achieve the purpose of improving the conversion rate of the raw material. The mother liquor after the impurity removal treatment can reach the purpose of increasing the application of the liquid and reducing the side reaction, thereby fulfilling the purpose of stabilizing production and improving yield further.

S3: the sodium taurate solution generated in S2 is prepared at a certain concentration, and the slurry of taurine can be generated by mixing through the acidic cation exchange resin column, and then concentrated and crystallized to generate crude taurine and mother liquor, and the separation temperature is about 25°C; or using sulfuric acid neutralization process, reaching the pH value of 7.0-8.5 by adding sulfuric acid, to generate taurine crystal solution; after cooling and crystallizing, the crude taurine and mother liquor could be generated, and the separation temperature is about 32°C -35°C.

S4: the mother liquor collected by S3 is further concentrated and crystallized, and the taurine is separated and extracted by means of the plate and frame and the like, and then the last mother liquor of taurine is concentrated.

S5: add a certain amount of liquid alkali to the last mother liquor of taurine collected in S4 and add the amount to 5-50% of the volume of the last mother liquor and stir it evenly. A certain amount of sulfuric acid is added further thereto to reach the pH value of 7.0-10.5, and the reaction temperature is controlled at 50°C - 95°C. The substance in the solution is present in the form of a sodium salt firstly, and then sulfuric acid is formed when sulfuric acid is added.

S6: transfer the material collected by S5 to a concentrator system for concentration, and the water is concentrated and evaporated by 30%-60%, and the times of concentration is about 1.4-2.5; control the concentration times and the concentration temperature to generate the best crystallization effect, and precipitate impurities and sodium sulfate.

S7: transfer the material collected by S6 to the cooling crystallization tank and cool down to 65-99°C, because the solubility of sodium sulfate will increase with the decrease of temperature, and the solubility of taurine will increase with the increase of temperature. To ensure that the impurities and sodium sulfate reach a certain ratio, and the taurine will not precipitate, and the high temperature also makes the viscosity of the solution lower down, because a certain amount of sodium sulfate crystals exist in the impurities, forming the filter bridge, facilitating the separation of impurities and then enters the plate frame filtering enables fast separation.

S8: the solution collected by S7 is cooled to 15-25°C, a certain amount of activated carbon is added, liquid alkali is added to adjust the pH value to 9.0-10.5, and the mother liquid is separated by a filter device such as a plate and frame or microporous filter;

S9: add the liquid ammonia into the taurine mother liquor which is cleaned of impurities thoroughly collected by S8 under the cooling condition until the ammonia content is higher than 15%, and a large amount of sulfate and other impurities will be precipitated, and then the blade filter or the sealed plate and frame is used. The clear mother liquor is filtered, and the generated mother liquor can be used in the aminolysis step (S2 step). The said filter device needs to comply with environmental protection requirements and must be sealed to prevent leakage of ammonia.

In order to explain the technical effects of this invention, the embodiments will be described below. All of the raw materials used in the following embodiments are commercially available unless otherwise specified. The methods are conventional unless otherwise specified, and the content of the materials refers to the mass percentage by volume unless otherwise specified.

### Embodiment 1

In this embodiment, the last mother liquor of taurine is treated with alkali and acid, and is decolorized and removed of impurity by activated carbon:
(1) The last mother liquor of taurine: 1500ml of taurine mother liquor, wherein the mass percentage is 10% (based on taurine, 100ml solution contains 10g of taurine), and 375ml of the liquid alkali concentration of 32% is added and stirred well, and then 100ml of concentrated sulfuric acid with the concentration of 98% is added, and the reaction temperature was controlled at 50-75°C.
(2) The said solution is concentrated to 1185ml, and the concentrated liquor is suction filtration at 75°C, and the time taken for filtration is 15min (minutes). After filtration, 975ml of the filtrate and 510g of the solid could be generated. The solid is relatively dry and the share of water is 15%.
(3) 46ml of the liquid alkali with concentration of 32% is added to the said filtrate, and the temperature is lowered to 18-22°C, and then 1g of activated carbon is added thereto. After stirring for a certain period of time, 970 ml of the mother liquid is generated by suction filtration. The main components are taurine and sodium isethionate. The testing content of taurine is 15.2% and the content of sodium isethionate is 18.2%. Compared with the last mother liquor of taurine before treatment, the content of taurine and sodium isethionate is obviously increased, and the content of impurities such as ethylene glycol and Fe are significantly reduced.

The testing data is as follows:

| Item | The last mother liquor of taurine (before treatment) | The last mother liquor of taurine (after treatment) |
|---|---|---|
| Ethylene Glycol | 6% | 0.5% |
| Fe | 10ppm | <1ppm |
| Content of Taurine | 10% | 15.2% |
| Content of Sodium Isethionate | 12% | 18.2% |
| Outer Appearance | Yellow | Light Yellow |

### Embodiment 2

This embodiment shows the control processing experiment of the last mother liquor of taurine:
(1) The last mother liquor of taurine: 1500ml of taurine mother liquor, wherein 10% by mass (based on taurine, 10g of taurine in 100ml of solution), the solution is concentrated to 1185ml. Then, the concentrated liquor is suction filtration at 75°C. When the filtration time is 3 hours, the solid matter generated by suction filtration is thinner, more viscous, with more water, and the moisture content is 30% (mass%); the suction filtration effect is not as good as embodiment 1, requiring the suction filter equipment of more power, higher energy consumption and lower efficiency.
(2) The last mother liquor of taurine: 1500ml of taurine mother liquor, wherein 10% by mass (calculated as taurine, 10g of taurine in 100ml of solution), the solution was concentrated to 1185ml. The concentrated liquor was separated at 75°C by a centrifuge, and it shall be centrifuged for 15 minutes, and the solid generated by centrifugation is relatively thinner, more viscous, with more water, and the moisture content is 28% (mass%), mainly subject to the higher viscous concentrate. The viscosity is higher and the separation effect is poor.

### Embodiment 3

Conduct ammonia-mixing treatment to the last mother liquor in Embodiment 1 and recover it.

The ammonia-mixing treatment of the recovered mother liquid: the mother liquid collected in Embodiment 1 is injected liquid ammonia to reach the content of 15% - 20% and filtered to obtain a transparent clear solution.

Preparation of sodium taurine: when the catalyst is present as sodium hydroxide, the sodium isethionate solution (manufactured by the Company), the ammonia-mixing treated mother liquor and the ammonia gas are subjected to an aminolysis reaction, and the aminolysis reaction is carried out at 220-280°C and 10-15MPa. The reaction is carried out for 1 hour. After the reaction is completed, the solution generated by flashing off the ammonia gas is the sodium taurate solution.

Through testing, the treated mother liquor is substantially free of sulfate.

### Embodiment 4

Conduct directly ammonia-mixing treatment on the last mother liquor before the treatment in Embodiment 1, and the step S7 is for recycling.

The ammonia-mixing treatment of the recovered mother liquor: the raw material in Embodiment 1 (i.e., the last mother liquor before the treatment) was passed through the liquid ammonia to reach the content of 15%-20% and filtered to obtain a transparent clear solution.

Preparation of sodium taurate: when the catalyst is present as sodium hydroxide, the sodium isethionate solution (manufactured by the Company), the ammonia-mixing treated mother liquor and the ammonia gas are subjected to an aminolysis reaction, and the aminolysis reaction is carried out at 220-280°C and 10-15MPa. The reaction is carried out for 1 hour. After the reaction is completed, the solution generated by flashing off the ammonia gas is the sodium taurate solution.

### Embodiment 5

Two sets of Embodiments and the corresponding comparative embodiments are selected to show the situation of aminolysis and the subsequent extraction of crude taurine under various conditions of mother liquor reuse;

All the following embodiments are 1.5 moles of sodium isethionate, and then the sodium taurate solution is prepared according to the methods said in Embodiments 3 and 4. The generated sodium taurate solution is treated with a cation exchange resin to obtain taurine. The solution is concentrated, and the taurine solution is concentrated and cooled to obtain a crude taurine, and the content of taurine is detected.

Calculate the yield ratio according to the following formula:

| SN of Experime nt | Item | Mother Liquor | | | Sodium isethiona te | Sodium taurate solution | Ammonia yield ratio | Crude taurine | |
|---|---|---|---|---|---|---|---|---|---|
| | | Volume ml | Taurine content (g/ml) | Sodium isethionate content (g/ml) | Mass (g) | The quality of pure taurine (g) | Calculating by the sodium isethionate input | Mass (g) | Content (g/g) |
| 1 | Prior to treatment of Embodiment 4 | 115 | 10% | 12% | 222 | 172.5 | 92.00% | 162.7 | 88% |
| 2 | Prior to treatment of Embodiment 4 | 135 | 10% | 12% | 222 | 177.2 | 94.50% | 171 | 86% |
| 3 | After treatment of Embodiment 3 | 75 | 15.20% | 18.20% | 222 | 188.44 | 100.00% | 173.2 | 93.10% |
| 4 | After treatment of Embodiment 3 | 90 | 15.20% | 18.20% | 222 | 206.63 | 110.00% | 188.5 | 93.20% |

Thereinto, Ammonia yield ratio = the mass of pure taurine (Mass of sodium isethionate:148 x 125) x 100%.

By comparing Experiment 3 and Experiment 4 in Embodiment 5, the increased dosage of the treated mother liquor increases the yield of the aminolysis reaction, while the crude product content is not affected, and the mother liquor impurities after the counter-treatment would be less.

Comparing Experiment 1 and Experiment 3 , and comparing Experiment 2 with Experiment 4 in Embodiment 5, the amount of mother liquor before and after treatment is the same under the same conditions (the same amount of purity), the yield of aminolysis reaction increases significantly, and the content of the crude product is significantly improved, which fully demonstrates that the mother liquor is more thoroughly reduced after treatment, so that the side reaction under the aminolysis reaction condition is greatly refrained, and the product quality is greatly improved.

Comparing the experiments 1, 2, 3, and 4 in Embodiment 5 overall, the effective content of the mother liquor after treatment is greatly improved, and at the same time, it is very obvious to improve the yield and increase the crude product content, and at the same time, the mother liquor can obviously increase the amount of the sleeve after the treatment (under the situation that the pureness is equivalent).

### Embodiment 6

In this embodiment, salting treatment and activated carbon decolorization and impurity removal treatment are conducted on the last mother liquor of taurine:
(1) The last mother liquor of taurine: 1500ml of taurine mother liquor, wherein 10% by mass (calculating according to taurine, 10g of taurine in 100ml of solution), and 127.8 g of Na₂SO₄ salt (i.e. 0.9mol), stir it evenly, and control temperature at 50-75°C.
(2) The said solution is concentrated to 1185ml, and the concentrated liquor is suction filtration at 75°C, and the time taken for filtration is 15 minutes. After filtration, 1050ml of the filtrate and 210g of the solid could be generated. The solid is relatively dry and the share of water is 23%.
(3) 50ml of liquid alkali with a concentration of 32% is added to the said filtrate, and the temperature is lowered to 18-22°C, and then 1g of activated carbon is added. After stirring for a certain period of time, 1046ml of the mother liquid is generated by suction filtration.

The main components are taurine and sodium isethionate. The testing content of taurine is 13.8% and the content of sodium isethionate is 16.5%. Compared with the last mother liquor of taurine before treatment, the content of taurine and sodium isethionate is obviously improved, and the content of impurities such as ethylene glycol and Fe are significantly reduced.

The testing data is as follows:

| Item | The last mother liquor of taurine (before treatment) | The last mother liquor of taurine (after treatment) |
|---|---|---|
| Ethylene Glycol | 6% | 0.8% |
| Fe | 10ppm | <1ppm |
| Content of Taurine | 10% | 13.8% |
| Content of Sodium Isethionate | 12% | 16.5% |
| Outer Appearance | Yellow | Light Yellow |

### Embodiment 7

In this embodiment, salting treatment and activated carbon decolorization and impurity removal treatment is conducted on the last mother liquor of taurine:
(1) The last mother liquor of taurine: 1500ml of taurine mother liquor, wherein the mass percentage is 10% (calculating according to taurine, 100ml solution contains 10g of taurine), and 568g of *Na₂SO₄* salt is added thereto (i.e. 4mol), stir it evenly, and control the temperature at 50-75°C.
(2) The said solution is concentrated to 1185ml, and the concentrated liquor is suction filtered at 75°C, and the time taken for filtration is 15 minutes. After filtration, 985ml of the filtrate and 774g of the solid could be generated. The solid is relatively dry and the share of water is 15%.
(3) 47ml of a liquid alkali with a concentration of 32% is added to the said filtrate, and the temperature is lowered to 18-22°C, and then 1g of activated carbon is added. After stirring it for a certain period of time, 980ml of the mother liquid is generated by suction filtration. The main components are taurine and sodium isethionate. The testing content of taurine is 14.8% and the content of sodium isethionate is 17.8%. Compared with the last mother liquor of taurine before treatment, the content of taurine and sodium isethionate is obviously improved, and the content of impurities such as ethylene glycol and Fe are significantly reduced.

The testing data is as follows:

| Item | The last mother liquor of taurine (before treatment) | The last mother liquor of taurine (after treatment) |
|---|---|---|
| Ethylene glycol | 6% | 0.6% |
| Fe | 10ppm | <1ppm |
| Content of Taurine | 10% | 14.8% |
| Content of Sodium Isethionate | 12% | 17.8% |
| Outer Appearance | Yellow | Light Yellow |

The processing equipment will be described in detail below with reference to the attached drawings.

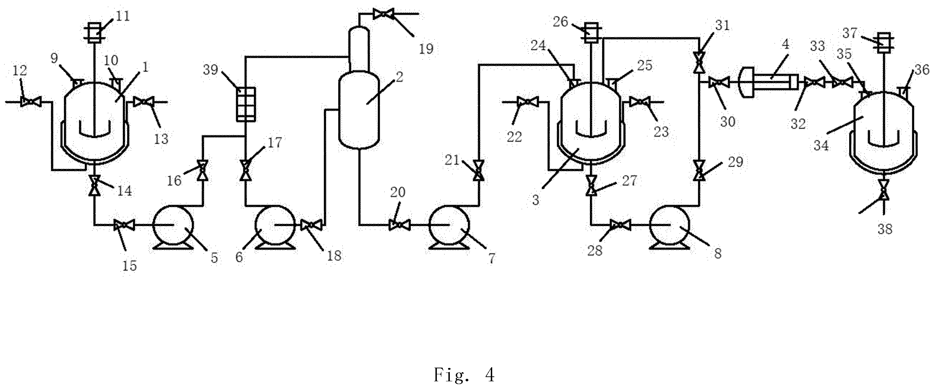

Figure 4 shows a pretreatment apparatus for removing the impurities and recovering taurine mother liquor. Utilizing the impurity removal and recovery method shown in Figure 1, which comprises sequentially tank 1 for adding alkali and acid, concentrator tank 2, concentrated and discharged crystal slurry tank 3, filter of plate and frame 4 and intermediate transfer tank 34 , and these instruments are connected, and the necessary equipment such as pumps are connected between the equipment, and the concentrator tank 2 is connected with a heat exchanger 39, and there are cooling mechanism on the alkali and acid adding tank 1 and the concentrated discharge slurry tank 3 for reducing the temperature of the material in the tank. Among them, the alkali and acid adding tank 1, the concentrated discharge crystal slurry tank 3 and the intermediate transfer tank 34 are all ordinary pressure equipment.

Wherein, the feed port 9 of the alkali and acid adding tank 1 is connected to the discharge port of the last mother liquor of taurine generated in the previous step. The mother liquid feed port 9, the exhaust port 10 and stirring mechanism 11 are installed on the alkali and acid adding tank 1, and the discharge valve 14 is arranged below the alkali and acid adding tank 1; a water circulation condensation layer is arranged outside the alkali and acid adding tank 1 for reducing the temperature in the alkali and acid adding tank 1. This frozen layer is provided with a cooling water inlet valve 12 and a cooling water outlet valve 13, and raw material pumps 5 and 6 are set between the concentration tank 2 and acid & alkali adding tank 1. Raw material pump 5 is equipped with feed valve 15 and discharge valve 16, and raw material pump 6 is equipped with feed valve 17 and discharge valve 18. Among them, the discharge valve 16 and feed valve 17 are connected with the heat exchanger 39. Above the concentration tank 2, the vent valve 19 (valve normally open) is equipped, and the lower part of the concentration tank 2 is connected to the raw material pump 7. The raw material pump 7 is provided with the feed valve 20 and the discharge valve 21, the feed valve 21 is connected with the feed port 24 of the concentrated discharge crystallizer tank 3. The mother liquor feed port 24, vent port 25 and rabbling mechanism 26 are equipped at the top of the concentrated discharge crystallizer tank 3, and the discharge valve 27 is installed below; the water-cycling frozen layer is equipped on the outer shell of the concentrated slurry tank 3 to lower down the temperature in the tank. Cooling water inlet valves 22 and 23 are installed in this interlayer, which is connected with plate and frame filter pump 8 through the feed valve 28 of the plate and frame filter pump. The discharge valve 29 and reflux valve 31 are installed in plate and frame filter pump 8. The inlet and outlet of plate and frame filter pump 4 are equipped with feed valve 30 and discharge valve 32, which are connected, through the feed valves 33 of transit tank, with the transit tank 34. The feed port 35, vent port 36 and rabbling mechanism 37 are installed on the top of transit tank 34, and the discharge valve 38 at the bottom is connected with the subsequent equipment. Apart from the said pipeline valves, there are some necessary valves and connecting parts for the manufacturing equipment, which are the common technological means and would not be described in detail herein.

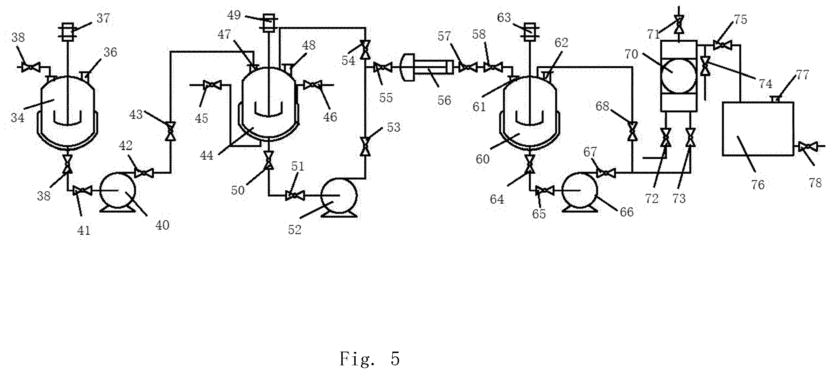

Figure 5 displays the preferred subsequent device for impunity-removal, namely the material flowing out of transit tank 34 entering into the activated carbon decolorization and removing impurities device. In other words, the last mother liquor of taurine generated by the pretreatment in the previous step is subjected to decolorization and removing impurities by activated carbon. Specifically, the activated carbon decolorizing and removing impurities device includes the decolorizing tank 44, the inlet frame filter pump 52, the plate and frame filter 56, the transit tank 60, the precision filter pump 66, the precision filter 70, and the receiving storage tank 76, which are sequentially connected. The precision filter 70 has a smaller filter pore size than the plate and frame filter 56 and can filter out small particle impurities. Among them, the decolorization tank 44, the intermediate tank 60 and the receiving storage tank 76 are all ordinary pressure equipment.

The exterior shell of the decolorization tank 44 is equipped with a water circulation condensation layer for reducing the temperature inside the tank of the decolorization tank 44. The interlayer is provided with the cooling water inlet valve 45 and the cooling water outlet valve 46. The decolorization tank 44 is equipped with the stirring mechanism 49, and the upper portion of the tank 44 is equipped with a mother liquid feeding port 47 and the exhaust port 48, the tank 44 is provided with the discharge valve 50 at the bottom. The discharge valve 50 is connected with the plate and frame filter pump 52 through the feeding valve 51 of the inlet frame filter pump 52, and the plate and frame filter pump 52 is provided with a discharge valve 53 and the return valve 54; and the inlet and outlet of the plate and frame filter 56 are respectively provided with a feed valve 55 and a discharge valve 57 and are connected to the intermediate transfer tank 60 through a feed tank inlet valve 58. The feed port 61, the port 62 and the agitation mechanism 63 are installed above the transit tank 60, the bottom discharge valve 64 is connected to the precision filter pump feed valve 65, the outlet end of the precision filter pump 66 is connected to the discharge valve 67, and connects with the precision filter pump return valve 68 and precision filter feed valve 73. The precision filter 70 is equipped with the cleaning water inlet valve 71, the cleaning water outlet valve 72 and the exhaust valve 74. The discharge valve 75 of the precision filter 70 is connected to the inlet of the receiving storage tank 76. The receiving storage tank 76 is equipped with the exhaust port 77, and its discharge valve 78 is connected to the subsequent devices. Other valves and connecting components necessary for the manufacturing equipment are common technical means in the art and will not be described herein. A liquid feed port is preferably installed on the bleaching tank for adding and filling the liquid alkali or the liquid acid.

After the device is added, the filtration efficiency and the processing capacity can be improved by two-stage filtration. Operation method: the activated carbon is added from the mother liquid feeding port 47, and can also be added from other openings, which is not limited. The discharge valve 50 of plate frame filtering decolorizing tank is closed, and the material is added to the decolorizing tank 44 through feed port 47 of the decolorizing tank. The cooling water outlet valve 46 and the cooling water inlet valve 45 are open, lower down the temperature by the cooling water, meanwhile, the decolorizer stirring mechanism 49 is started. After the temperature is lowered to the predetermined temperature, the cooling water inlet valve 45 and the cooling water outlet valve 46 are closed. The decolorizer discharge valve 50, the inlet frame filter pump feed valve 51, the plate and frame feed valve 55, the plate and frame discharge valve 57 and the transit tank feed valve 58 are open, and the transit tank discharge valve 64 is closed to start the plate and frame filter pump 52, then adjust the pressure of the inlet frame through the plate and frame filter pump return valve 54. The activated carbon and the material are introduced into the frame filter 56, and the activated carbon is trapped in the filter 56, open the plate and frame, the activated carbon that has adsorbed impurities could be discharged. After the material of the transit tank 60 reaches a certain volume, the transit tank discharge valve 64, the precision filter pump feed valve 65, the precision filter pump return valve 68, the precision filter feed valve 73, the precision filter exhaust valve 74 and the precision filter discharge valve 75 are opened, and the precision filter pump 66 is launched, and the precision filter pump discharge valve 67 is opened. After the precision filter exhaust valve 74 is discharged, the precision filter vent valve 74 is closed. The feed pressure of the precision filter 70 is adjusted by the precision filter pump return valve 68. The material collected by the receiving tank 76 is sent to a subsequent section for processing.

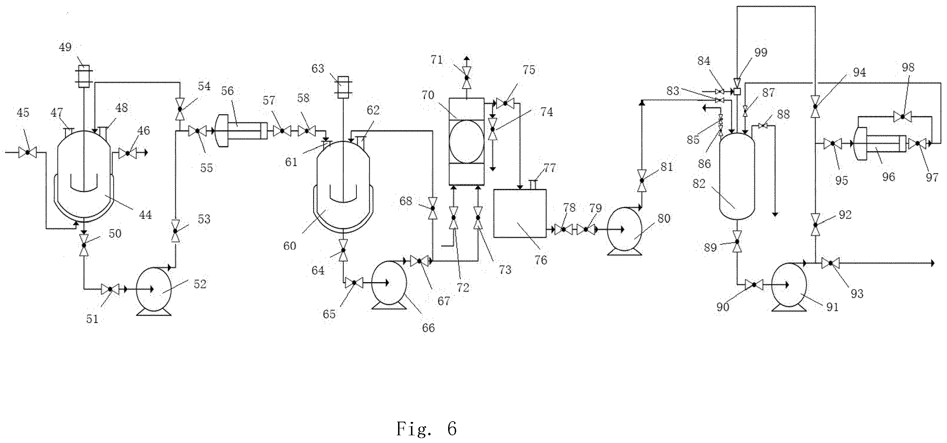

An optimized process and equipment is shown in Figure 6 and comprises an activated carbon decolorization and removing impurity device and an ammonia-mixing and desalination device. The ammonia-mixing and desalination device includes flow-through ammonia-mixing reaction tank 82, pump 91, and vane filter 96, which are sequentially connected, and the vane filter 96 can also be replaced with a sealed plate and frame filter. The ammonia-mixing reaction tank 82, the ammonia aspirator 99, the ammonia inlet valve 84, the feed valve 83, the clear liquid returning tank valve 87 and an emptying valve 88, and the safety valve 86 and the safety valve front control valve 85 are equipped above the ammonia-mixing reaction tank, and the ammonia reaction is carried out. The bottom of the ammonia-mixing reaction tank is equipped with the discharge valve 89, and the discharge valve 89 is connected with the pump feed valve 90. The other end of the pump 91 is respectively equipped with the pump discharge valve 92, the rotary discharge valve 93. The outlet direction of the pump discharge valve 92 is connected to the ammonia aspirator 99 through the inlet vane reflux valve 94 and is connected to the vane filter 96 through the vane machine feed valve 95. The vane filter is equipped with the overflow valve 98 and the discharge valve 97, and the discharge valve 97 is connected with the clear liquid reflux tank valve 87. Similarly, there are some necessary valves and connecting components for the manufacturing equipment, which are common technical means in the art and would not be described herein.

The said ammonia-mixing desalination device can remove salt and impurities in the mother liquor, and the operation process is simple. Operation method: open the front control valve 85 of the safety valve, open the emptying valve 88, open the feed valve 83 to add mother liquid to the ammonia-mixing reactor 82, and close the feed valve 83 after finishing feeding. Open the discharge valve 89 at the bottom, pump feed valve 90, inlet vane return valve 94, start pump 91, open pump discharge valve 92, stabilize and then open ammonia entering valve 84, and close the emptying valve 88, absorb ammonia to reach the content of 15% (mass to volume ratio, 15g/100ml) or more, then stop absorption, and the ammonia inlet valve 75 is closed; the blade machine overflow valve 98, the supernatant reflux tank valve 87, and the vane machine feed valve 95 are open. After the filter 96 is full, the vane machine discharge valve 97 is opened and the vane machine overflow valve 98 is closed at the same time. During the cycle, samples are taken to observe the state of the material in the ammonia-mixing reactor 96 until the filtrate is clear. After the filtrate is clear, the transit and discharge valve 93 can be opened to transfer the material to the aminolysis reaction step.

## Claims

1. A method for fully recovering the taurine mother liquor in the ethylene oxide production process of taurine, comprising the steps:
(a) adding alkali firstly and then acid to a last mother liquor of taurine to form salt in the solution, or directly adding salt formed by the reaction of alkali with acid to the last mother liquor of taurine, wherein the alkali is any one or the mixture of sodium hydroxide, potassium hydroxide, lithium hydroxide, sodium carbonate, potassium carbonate and lithium carbonate, and the acid is sulfuric acid, hydrochloric acid, or an organic acid;
(b) concentrating and crystallizing the material collected in step (a); and
(c) obtaining a clear last mother liquor of taurine through filtration.

2. The method according to claim 1, wherein the reaction temperature range of step (a) is from 50°C-95°C, and the pH value after acid addition is between 7.0-10.5.

3. The method according to claim 2, wherein the amount of alkali added in step (a) is 5% - 50% of volume of the said taurine last mother liquor.

4. The method according to claim 1, wherein the liquid alkali and sulfuric acid are added in step (a) to form sodium taurine and sodium sulfate.

5. The method according to claim 1, wherein 30%-60% water is condensed and evaporated in step (b).

6. The method according to claim 1, wherein 530 mol - 5300 mol salt is added in step (a) to 1 m³ of the said taurine last mother liquor.

7. The method according to anyone of the preceding claims, further comprising the step: adding activated carbon to the said taurine last mother liquor generated by step (c) for decolorization under cooling conditions, and lower down the pH value to 9.0 to 10.5 by adding alkali, and then filtering.

8. The method according to claim 7, wherein the cooling condition refers to that the temperature in production system is lower than the temperature of the previous process, and the treatment temperature of system is controlled among 15-25°C.

9. The method according to claim 8, wherein the liquid at the outlet after the process of the activated carbon decolorization and alkali filtration is subjected to ammonia-mixing treatment, and the mother liquid removed of impurity is generated by solid-liquid separation, and the mother liquid is all returned to the aminolysis step of the ethylene oxide production process.

10. The method according to claim 9, wherein during the said ammonia-mixing treatment, the liquid ammonia or ammonia gas is added to the said outlet liquid, and the mass-to-volume ratio of ammonia is 15 g/100 ml or more.

## Patentansprüche

1. Verfahren zur vollständigen Rückgewinnung der Mutterlauge von Taurin in dem Ethylenoxid-Produktionsprozess von Taurin, umfassend die folgenden Schritte:
(a) Zugeben von zuerst Alkali und dann Säure zu einer letzten Mutterlauge von Taurin zur Ausbildung eines Salzes in der Lösung, oder direktes Zugeben des durch die Reaktion von Alkali mit Säure ausgebildeten Salzes zu der letzten Mutterlauge von Taurin, wobei das Alkali irgendeines oder das Gemisch ist von Natriumhydroxid, Kaliumhydroxid, Lithiumhydroxid, Natriumcarbonat, Kaliumcarbonat und Lithiumcarbonat, und die Säure Schwefelsäure, Salzsäure oder eine organische Säure ist;
(b) Konzentrieren und Kristallisieren des in Schritt (a) gesammelten Materials; und
(c) Erhalten einer klaren letzten Mutterlauge von Taurin durch Filtration.

2. Verfahren nach Anspruch 1, wobei der Bereich der Reaktionstemperatur aus Schritt (a) von 50 °C bis 95 °C beträgt und der pH-Wert nach Säurezugabe zwischen 7,0 und 10,5 beträgt.

3. Verfahren nach Anspruch 2, wobei die Menge an in Schritt (a) zugegebenem Alkali 5 bis 50 Volumen-% der letzten Mutterlauge von Taurin beträgt.

4. Verfahren nach Anspruch 1, wobei das flüssige Alkali und die flüssige Schwefelsäure in Schritt (a) zur Ausbildung von Natriumtaurin und Natriumsulfat zugegeben werden.

5. Verfahren nach Anspruch 1, wobei in Schritt (b) 30 % bis 60 % Wasser kondensiert und verdampft werden.

6. Verfahren nach Anspruch 1, wobei in Schritt (a) 530 mol bis 5300 mol Salz zu 1 m³ der letzten Mutterlauge von Taurin gegeben werden.

7. Verfahren nach jedem der vorangehenden Ansprüche, weiterhin umfassend den folgenden Schritt: Zugeben von Aktivkohle zu der durch Schritt (c) erzeugten letzten Mutterlauge von Taurin zur Entfärbung unter Kühlbedingungen, und Absenken des pH-Werts auf 9,0 bis 10,5 durch Zugeben von Alkali, und dann Filtrieren.

8. Verfahren nach Anspruch 7, wobei sich die Kühlbedingung darauf bezieht, dass die Temperatur im Produktionssystem niedriger ist als die Temperatur des vorausgegangenen Prozesses, wobei die Behandlungstemperatur im System auf zwischen 15 und 25 °C geregelt wird.

9. Verfahren nach Anspruch 8, wobei die Flüssigkeit am Auslass nach dem Prozess der Entfärbung mit Aktivkohle und der Alkalifiltration einer Mischbehandlung mit Ammoniak unterzogen wird, und die von Unreinheiten befreite Mutterlauge durch Fest-Flüssig-Trennung erzeugt wird und die gesamte Mutterlauge zu dem Aminolyseschritt des Ethylenoxid-Produktionsprozesses zurückgeführt wird.

10. Verfahren nach Anspruch 9, wobei während der Mischbehandlung mit Ammoniak das flüssige Ammoniak oder Ammoniakgas zu der Flüssigkeit am Auslass gegeben wird, wobei das Masse zu Volumen-Verhältnis von Ammoniak 15 g/100 ml oder mehr beträgt.

## Revendications

1. Procédé de récupération complète de liqueur de taurine dans le processus de production d'oxyde d'éthylène de la taurine, comprenant les étapes consistant à :
(a) ajouter un alcali premièrement puis de l'acide à une dernière liqueur mère de taurine pour former un sel dans la solution ou ajouter directement le sel formé par la réaction de l'alcali avec l'acide à la dernière liqueur mère de taurine, l'alcali étant l'un quelconque ou le mélange de l'hydroxyde de sodium, de l'hydroxyde de potassium, de l'hydroxyde de lithium, du carbonate de sodium, du carbonate de potassium et du carbonate de lithium, et l'acide étant l'acide sulfurique, l'acide chlorhydrique ou un acide organique ;
(b) concentrer et cristalliser le matériau collecté dans l'étape (a) ; et
(c) obtenir une dernière liqueur mère de taurine claire par filtration.

2. Procédé selon la revendication 1, dans lequel la plage de température réactionnelle de l'étape (a) est de 50 °C à 95 °C et la valeur du pH après l'addition de l'acide est comprise entre 7,0 à 10,5.

3. Procédé selon la revendication 2, dans lequel la quantité d'alcali ajoutée dans l'étape (a) est de 5 % à 50 % du volume de ladite dernière liqueur mère de taurine.

4. Procédé selon la revendication 1, dans lequel l'alcali liquide et l'acide sulfurique sont ajoutés dans l'étape (a) pour former de la taurine de sodium et du sulfate de sodium.

5. Procédé selon la revendication 1, dans lequel 30 % à 60 % de l'eau est condensée et évaporée dans l'étape (b).

6. Procédé selon la revendication 1, dans lequel 530 moles à 5300 moles de sel sont ajoutées dans l'étape (a) à 1 m³ de ladite dernière liqueur mère de taurine.

7. Procédé selon l'une quelconque des revendications précédentes, comprenant en outre l'étape consistant à :
ajouter du charbon actif à ladite dernière liqueur mère de taurine produite par l'étape (c) pour la décoloration dans des conditions de refroidissement, et abaisser la valeur du pH de 9,0 à 10,5 en ajoutant un alcali, puis filtrer.

8. Procédé selon la revendication 7, dans lequel la condition de refroidissement se rapporte à celle où la température dans le système de production est plus basse que la température du processus précédent, et la température de traitement du système est contrôlé autour de 15 à 25 °C.

9. Procédé selon la revendication 8, dans lequel le liquide à la sortie après le processus de décoloration par charbon actif et la filtration d'alcali est soumis à un traitement de mélange d'ammoniaque et la liqueur mère aux impuretés enlevées est produite par séparation solide-liquide, et la liqueur mère est retournée entièrement à l'étape d'aminolyse du processus de production d'oxyde d'éthylène.

10. Procédé selon la revendication 9, dans lequel durant le traitement dudit mélange d'ammoniaque, l'ammoniaque liquide ou le gaz ammoniac est ajouté audit liquide de sortie et le rapport de masse à volume de l'ammoniaque est de 15 g/100 ml ou plus.
